# EUROPEAN PATENT SPECIFICATION

(11) **EP 3 308 698 B1**
(45) Date of publication and mention of the grant of the patent: **26.02.2020**
(21) Application number: 16306359.7
(22) Date of filing: 14.10.2016
(51) Int. Cl.: A61B 5/00, A61N 1/378, A61B 5/07, H02J 5/00, H02J 7/02, H04B 5/00, H01F 3/10

(54) **PROBE AND METHOD OF OPERATING A PROBE**
SONDE UND VERFAHREN ZUM BETRIEB EINER SONDE
SONDE ET PROCÉDÉ DE FONCTIONNEMENT D'UNE SONDE

(43) Date of publication of application: 18.04.2018
(73) Proprietor: Alcatel Lucent, 91620 Nozay (FR)
(72) Inventor: Wünstel, Klaus, 70435 Stuttgart (DE); Banniza,Thomas-Rolf, 70435 Stuttgart (DE)
(74) Representative: DREISS Patentanwälte PartG mbB

(56) References cited:
- WO-A2-2016/010772
- US-A1- 2009 167 449
- US-A1- 2009 174 400
- US-A1- 2011 304 220

## Description

### Field of the invention

The disclosure relates to a probe for use in the human and/or animal body. The disclosure further relates to a method of operating such a probe and to a system comprising at least one probe of the aforementioned type. Herein, probe means a device that may be placed in the body of a human or animal.

### Background

Conventional probes may e.g. be introduced into a human or animal body, e.g. into the gastro-intestinal (GI) tract of the human or the animal to measure certain physical parameters in the animal or human body. Disadvantageously, the conventional probes typically use radio frequency, RF, signals for communication with a device external to the body, and such RF communication may require comparatively large antennas for transmitting the RF signals. Further disadvantageously, the conventional probes are battery-powered, i.e. comprising a comparatively large installation space for the battery, while having a rather limited operating time. These factors prevent further miniaturization of the conventional probes and limit the usability to few fields of application.

US 2011/0304220 A1 discloses an inductive power transfer apparatus for a non-contact movement of at least one magnetic body. The body is arranged in a primary magnetic field of a permanent magnet which moves in a defined manner. Further a magnetic field sensor is provided to record a secondary magnetic field of the magnetic body.

WO 2016/010772 A1 refers to wireless power transfer using a transmitter that generates a dynamic magnetic field and receiver comprising a magnet and coil. In operation, the magnet rotates in response to the dynamic magnetic field and induces a voltage across the coil.

### Summary

The invention is defined by appended claims 1-12. Various examples provide an apparatus for use in human or animal body, comprising an improved probe, which is more flexible in use and does not suffer from one or more the abovementioned limitations.

The probe comprises a housing, at least one element having a permanent magnetic dipole moment, and an electrical circuit, the element having a permanent magnetic dipole moment being rotatable within said housing, the element having a permanent magnetic dipole moment being configured to generate an electromotive force in said electrical circuit in response to being rotated.

This configuration advantageously enables to supply the probe with energy from an external source, e.g. by driving a rotating movement of the element having a permanent magnetic dipole moment using magnetic fields generated and/or controlled by said external source. At the same time, exchange of information between the probe or a component thereof and an external device is enabled, as will be explained in detail further below.

The probe does not necessarily require a local storage medium for electric energy such as a battery or the like, as energy may be provided from the external source just in time, e.g. when required.

Also, the magnetic fields generated and/or controlled by said external source are not substantially attenuated by the human or animal body so that an efficient energy transfer (and also communication) is enabled between the external source and the probe even if the probe is positioned within body tissue.

Although the probe is particularly well-suited for use in the human or animal body, according to further embodiments, it may also be used in other environments such as e.g. (biochemical) reactors, pipes, or generally any other target systems where measurements are to be made and/or where agents are to be deployed, especially if a direct mechanical contact to said probe is at least temporarily impossible. Also, as the principle according to the embodiments relies on magnetic fields to drive the rotatable element having a permanent magnetic dipole moment of the probe, energy transfer from an external device to said probe is nearly always possible, except with situations where a strong magnetic shielding is applied around the probe.

The electric circuit comprises at least one induction coil, as will be explained in further detail below.

According to further examples, the electrical circuit may comprise one or more electronic components such as e.g. a control unit, as will also be explained in further detail below.

According to an example, the element having a permanent magnetic dipole moment is rotatably attached to a shaft, wherein said shaft is e.g. fixedly attached to an inner wall of said housing of the probe or a suitable mechanical support structure provided within said housing. This represents a type of "internal bearing" for the element having a permanent magnetic dipole moment.

Alternatively or additionally, an external bearing may be provided for rotatably arranging said element having a permanent magnetic dipole moment within said housing of the probe.

According to an example, said element having a permanent magnetic dipole moment comprises at least one permanent magnet, wherein preferably a maximum length dimension of said permanent magnet is smaller than about 15 millimeter (mm), more preferably smaller than about 1.5 mm, which enables a particularly small configuration.

According to an example, said element having a permanent magnetic dipole moment or permanent magnet, respectively, may comprise or consist of a bar magnet, wherein said bar magnet may substantially comprise cuboid shape with a width a, a length b, and a depth c, wherein e.g. b > a and b > c, and wherein said length b e.g. corresponds to the abovementioned maximum length dimension, wherein preferably b < 15 mm, or more preferably b < 1.5mm. These dimensions enable a very compact structure for the complete probe, so that efficient deployment e.g. in blood vessels and other regions of the human and/or animal body is possible, e.g. by injecting the probe to the target region.

According to Applicant's analysis, advantageously, further miniaturization or scaling, respectively, of the element having a permanent magnetic dipole moment and the complete probe comprising said element having a permanent magnetic dipole moment is also possible, wherein maximum length dimensions for the element having a permanent magnetic dipole of e.g. in the micrometer (pm) or even nanometer (nm) range may be attained. This enables to provide probes according to the embodiments with outer dimensions of e.g. few hundreds of micrometer or few hundreds of nanometer, thus providing new fields of application.

According to a further example, said element having a permanent magnetic dipole moment comprises a remanent magnetic flux density of at least about 0.1 Tesla, T, (1 T = (1 kg)/(A^{∗}s²)), preferably of at least about 1.4 Tesla, which enables to attain high driving torques even for elements having a permanent magnetic dipole moment with maximum length dimensions in the micrometer or nanometer range.

As an example, magnetic material of the NdFeB-type, i.e. comprising an alloy of neodymium (Nd), iron (Fe) and boron (B), e.g. Nd2Fe14B, may be used to form the element having a permanent magnetic dipole moment or components thereof.

According to some embodiments, the abovementioned bar magnet, which may be considered as a simple exemplary embodiment of a magnetic dipole, is particularly preferred due to its simplicity and commercial availability. However, according to further embodiments, other types of elements with non-vanishing magnetic dipole moments, may also be used within the probe.

According to a further example, said probe comprises an induction coil that is magnetically coupled with said element having a permanent magnetic dipole moment so that an electromotive force is produced in said induction coil by rotation of said element having a permanent magnetic dipole moment. Preferably, according to a further embodiment, a controllable electric resistor is connected in parallel with (i.e., "across") said induction coil.

This advantageously enables to influence locally (i.e., within the probe) the energy and movement of the rotating element having a permanent magnetic dipole moment, e.g. by dissipating a controllable amount of energy within the controllable electric resistor. An external magnetic "receiver" may monitor the magnetic field caused by the rotating element having a permanent magnetic dipole moment, whereby e.g. an amplitude and/or a frequency of an electric voltage induced into a receiver coil of the magnetic "receiver" may be determined. Variations in amplitude and/or frequency of said induced voltage may in a per se known manner be caused by extracting energy using the internal induction coil of the probe according to the present embodiment, such that by modulating the controllable resistor of the internal induction coil an externally detectable signal may be provided, which may be used for data communication between the probe and an external device connected to said external receiver coil.

Similarly, according to a further example, a transfer of information from an external device to the probe may be effected by controlling or varying the frequency and/or amplitude of the externally applied magnetic field.

In the present context, the magnetic coupling between the induction coil and the element having a permanent magnetic dipole moment of the probe means that said induction coil is configured and arranged relatively to said element having a permanent magnetic dipole moment such that at least a part of the magnetic flux of the element having a permanent magnetic dipole moment may at least temporarily (e.g., depending on an angular position of the rotatably arranged element having a permanent magnetic dipole moment) pass through said induction coil.

According to a further example, a control unit is provided in said housing, i.e. local to said probe. Said control unit may e.g. control an operation of the probe, for example by determining (e.g., by measuring) at least one of: a frequency and/or amplitude of an external magnetic field, a rotational speed of the element having a permanent magnetic dipole moment of the probe. The control unit may e.g. comprise an ASIC (application specific integrated circuit), a microcontroller, a digital signal processor (DSP), discrete logic elements, a programmable logic circuit or any combination thereof.

According to further examples, the probe may comprise one or more sensors, which may preferably at least partly be integrated into the housing of the probe, and which may be configured to measure one or more physical parameters of the probe and/or a medium surrounding the probe (i.e., temperature, pressure, pH value). In these embodiments, an optional control unit may evaluate sensor signals provided by said sensors. Also, the sensor signals or any data derived therefrom may be transmitted to an external device, e.g. by modulating the controllable electric resistor and thus an angular speed of the element having a permanent magnetic dipole moment as mentioned above.

According to a further example, said housing has a basically ellipsoidal or spherical shape, which facilitates movement or transport, respectively, of said probe in the target system (e.g. a gastrointestinal tract or a blood vessel or the like of a human being or an animal). However, according to further embodiments, polygonal or cuboid shapes may also be provided for the housing, especially in cases where it is preferred that the probe retains its position within the target system.

According to a further example, said probe comprises a device for: transforming mechanical rotational energy of said element having a permanent magnetic dipole moment into electrical energy, and/or into thermal energy, and/or into a flow of a fluid surrounding the housing of said probe and/or within the housing of said probe, and/or into propulsion energy for moving said probe.

According to a further example, it is also possible to provide within said probe a device for transforming said mechanical rotation energy of said element having a permanent magnetic dipole moment into acoustic, preferably ultrasonic, energy.

According to an example, the device for transforming mechanical rotational energy of said element having a permanent magnetic dipole moment into electrical energy may e.g. comprise an induction coil arranged locally in said probe, which induction coil is magnetically coupled to said element having a permanent magnetic dipole moment, similar to the embodiment with the induction coil and controllable resistor in parallel as explained above. Alternatively or additionally, said device for transforming mechanical rotation energy into electrical energy may comprise an electric generator which is directly mechanically driven by the rotating element having a permanent magnetic dipole moment. E.g., a shaft of the electric generator may be driven by the rotating element having a permanent magnetic dipole moment or a shaft of the rotating element having a permanent magnetic dipole moment.

According to a further example, said device for transforming mechanical rotational energy of said element having a permanent magnetic dipole moment into thermal energy may comprise a friction mechanism, wherein friction effects between the rotating element having a permanent magnetic dipole moment and at least one friction element contactable by the element having a permanent magnetic dipole moment cause transformation of rotational energy of the element having a permanent magnetic dipole moment into thermal energy.

According to a further example, the element having a permanent magnetic dipole moment may comprise a mass imbalance with respect to its axis of rotation so that a portion of its rotational energy may be transformed into vibrations, e.g. structure-borne noise.

According to a further example, the device for transforming mechanical rotational energy of said element having a permanent magnetic dipole moment into a flow of a fluid surrounding the housing of said probe and/or within the housing of said probe may comprise a propelling screw or the like effecting movement of a fluid outside of or inside of the probe. For example, a shaft of said element having a permanent magnetic dipole moment may be coupled to a propelling screw arranged at least partially outside of the housing of the probe. Alternatively or additionally, the probe may comprise at least one fluid channel which is in fluid communication with a fluid surrounding the probe, and in said fluid channel a propelling screw driven by the element having a permanent magnetic dipole moment may be provided.

According to a further example, said probe is configured to carry at least one agent, preferably a drug to be released within the human and/or animal body, and said probe is further configured to controllably release said agent, e.g. under control of a local control unit and/or under control of an external device transmitting corresponding control commands to the probe.

Some embodiments feature a method of operating a probe for use in the human and/or animal body according to claim 8.

According to an example, said probe comprises an induction coil that is magnetically coupled with said element having a permanent magnetic dipole moment, wherein said probe applies a predetermined magnetic damping to the element having a permanent magnetic dipole moment by controlling a resistance of a controllable electric resistor connected in parallel with said induction coil.

According to a another example, the application of said predetermined magnetic damping to the element having a permanent magnetic dipole moment is used for transmitting data from the probe to an external device.

According to a further example, said probe transforms mechanical rotation energy obtained from said element having a permanent magnetic dipole moment into at least one of: a) electrical energy, b) thermal energy, c) a flow of a fluid surrounding the housing of said probe and/or within the housing of said probe, d) propulsion energy for moving said probe, e) acoustic, preferably ultrasonic, energy.

Exemplary devices for attaining such transformation of energy types have already been explained above.

Yet further examples feature a system comprising at least one probe according to the embodiments and a magnetic field generator configured to provide a magnetic field in said at least one probe.

According to an example, said magnetic field generator may comprise at lease on pair of Helmholtz coils which may be arranged external to and preferably surrounding said at least one probe, providing a basically homogenous magnetic field between said pair of Helmholtz coils.

According to a further example, said magnetic field generator is configured to provide a substantially static magnetic field to align the element having a permanent magnetic dipole moment in a selected direction.

According to a further example, said magnetic field generator is configured to, in a first operational state, provide a temporally alternating magnetic field, particularly to drive a rotational movement of the element having a permanent magnetic dipole moment of said at least one probe, wherein optionally, said magnetic field generator is configured to, in a second operational state, provide a substantially static magnetic field, particularly to align the element having a permanent magnetic dipole moment of said at least one probe in a predetermined direction depending on said substantially static magnetic field.

For example, the first operational state may be employed to supply the probe with mechanical energy, while the second operational state may be used to align the element having a permanent magnetic dipole moment with the magnetic field provided in said second operational state, e.g. to establish a defined (optimum) starting position prior to entering the first operational mode.

According to an example, said system comprises a receiver configured to receive a magnetic signal from the element having a permanent magnetic dipole moment of said at least one probe. Said receiver may e.g. comprise a distinct magnetic coil capable of detecting a time variable magnetic flux generated by the rotating element having a permanent magnetic dipole moment of the probe (and modulations thereof that may be effected by damping the rotating element having a permanent magnetic dipole moment as explained above).

Alternatively, one or more coils of the magnetic field generator may also be used for detecting a time variable magnetic flux generated by the rotating element having a permanent magnetic dipole moment of the probe, e.g. in a time division duplexed manner with respect to generating a magnetic field for driving rotational movement of the element having a permanent magnetic dipole moment.

### Brief description of the figures

Further features, aspects and advantages of the illustrative examples are given in the following detailed description with reference to the drawings in which:
- Figure 1: schematically depicts a block diagram of a probe according to an example,

- Figure 2A and 2B: schematically depict block diagrams of a probe with an internal induction coil according to further examples,
- Figure 3A to 3C: schematically depict a probe with substantially spherical housing according to further examples,
- Figure 4A and 4B: schematically depict probes according to further examples,
- Figure 5: schematically depicts an operational scenario of a system according to an example including a probe configured for deployment in a human or animal body,
- Figure 6: schematically depicts a timing diagram of operational parameters of the system according to Figure 5,
- Figure 7A and 7B: schematically depict simplified flow-charts of a method of operating a probe according to further examples, e.g., for operating the probes of Figures 1 to 5, and
- Figure 8A and 8B: schematically depict simplified flow-charts of a method of operating a system including a probe, e.g., examples of the system of Figure 5.

### Detailed description

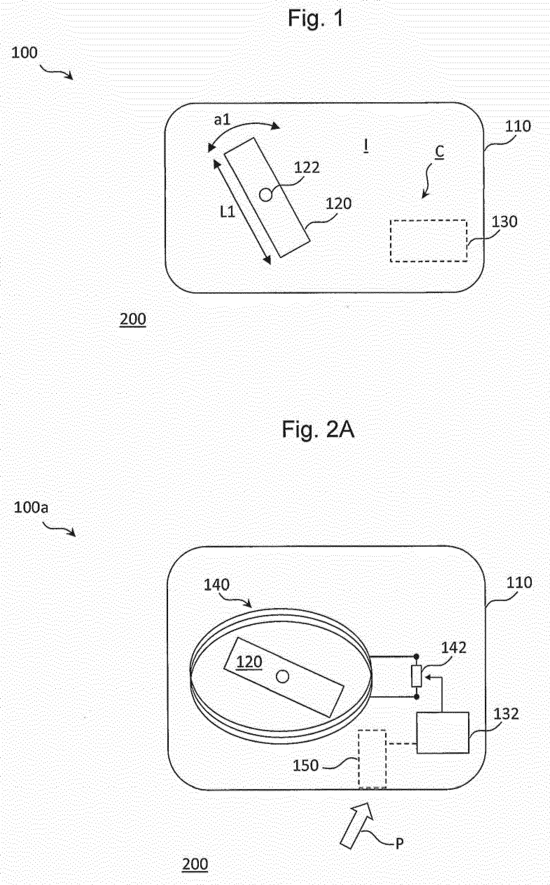

Figure 1 schematically depicts a block diagram of a probe 100 according to an example. The probe 100 is suitable for use e.g. in the human body or in an animal body, e.g. for the purpose of disease management, or remote doctor control. According to some embodiments, the probe may be used for releasing products such as medical agents (drugs) into the body and/or for monitoring local body function parameters and/or for performing manipulations of body parts, as will be explained in detail further below.

The probe 100 comprises a housing 110 and an element 120 having a permanent magnetic dipole moment. The element 120 having a permanent magnetic dipole moment is rotatably mounted within said housing 110, so that the element 120 having a permanent magnetic dipole moment may rotate, e.g., around an axis 122, as illustrated by the double arrow a1 of Fig. 1.

The housing 110 may be configured to protect the component(s) 120 in its interior I from influences of a surrounding medium (e.g., blood and/or body tissue), which may be present outside the housing, cf. reference numeral 200.

The probe 100 further comprises an electrical circuit C, and the element 120 having a permanent magnetic dipole moment is configured to generate an electromotive force (EMF) in said electrical circuit C in response to being rotated.

The probe 100 may advantageously enable to be wirelessly supplied with electrical energy from an external source (not shown). For example, the external source may magnetically drive a rotating movement of the element 120 having a permanent magnetic dipole moment via time-varying magnetic fields generated and/or controlled by said external source. In other words, by providing said time-varying magnetic fields from the external source, the element 120 having a permanent magnetic dipole moment within the probe may be set into rotation, which corresponds with a transfer of energy from said external magnetic field to rotational energy of said element 120 having a permanent magnetic dipole moment. Further, the rotation of the element 120 having a permanent magnetic dipole moment generates said EMF in the electric circuit C, as mentioned above.

According to some examples, this energy supply may be employed for wirelessly supplying the probe with electrical energy, e.g. by transforming the rotational energy of said element 120 having a permanent magnetic dipole moment at least partly into electrical energy within said probe, as stated above.

In some examples, the probe 100 may also wirelessly communicate with an external device, e.g., via the element 120 having a permanent magnetic dipole moment, as will be explained in detail further below.

Advantageously, in some examples, the probe 100 may not have or need a local storage medium for electric energy such as a battery or the like. Instead, operating energy may be provided from the external source via the element 120 having a permanent magnetic dipole moment, e.g., when the probe 100 is deployed in a human or animal body.

Typically, the magnetic fields generated and/or controlled by said external source would not be substantially attenuated by the human or animal body. Thus, an efficient energy transfer and/or possibly wireless communication is enabled between the external source and the probe 100, e.g., even when the probe 100 is positioned deeply within the human or animal body.

According to some examples, the probe may be used in other environments such as e.g. (biochemical) reactors, pipes, or another environment where a direct mechanical contact to said probe is at least temporarily impossible.

Also, some examples rely on magnetic fields of low frequency to operate and/or communicate with the probe 100.

For example, the external magnetic field may have a frequency in the range between about 0 Hz and about a few kHz, e.g., depending on the dimensions of the element 120 having a permanent magnetic dipole moment.

According to some examples, the element 120 having a permanent magnetic dipole moment is connected to the housing 110 via an elongated shaft, and the element 120 having a permanent magnetic dipole moment is able to rotate around an axial direction of said shaft (Fig. 1). The connection to the housing via such a shaft may be such that the shaft functions as a type of "mechanical bearing", particularly internal bearing, for the element 120 having a permanent magnetic dipole moment. Presently, a shaft may e.g. be provided at the position of the schematically indicated axis of rotation 122.

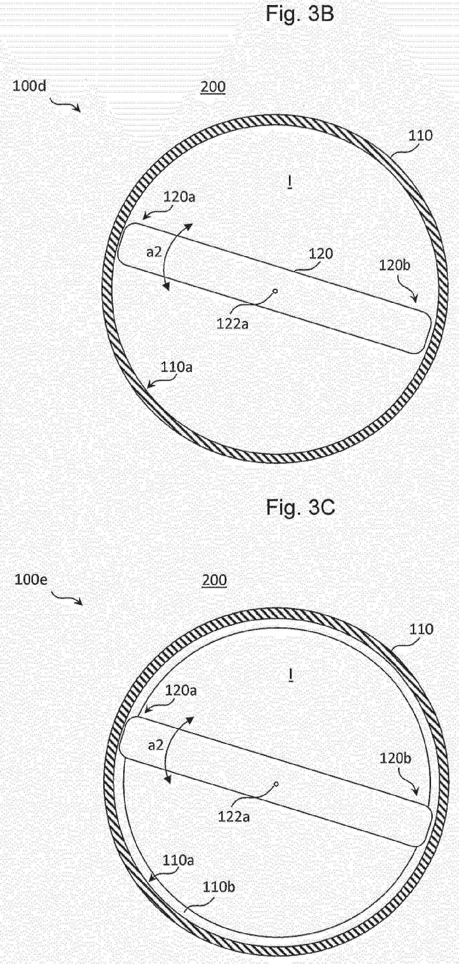

Alternatively or in addition to the internal bearing, the probe 100 may include an external bearing providing a low friction connection of the element 120 having a permanent magnetic dipole moment to the housing 110 thus enabling a rotational motion of the element 120 having a permanent magnetic dipole moment within the housing 110. For example, one or more axial end portions of said element 120 having a permanent magnetic dipole moment may be supported or guided within an annular groove provided e.g. on the inner surface of the housing device, also cf. the Fig. 3C embodiment further below.

According to an example, said element 120 having a permanent magnetic dipole moment comprises or consists of at least one permanent magnet, e.g. a bar magnet, cf. Fig. 1, wherein preferably a maximum length dimension L1 of said magnet is smaller than about 15 millimeter (mm), preferably smaller than about 1.5 mm. Such small linear sizes may enable a particularly small configuration for the probe 100. For example, said bar magnet may substantially comprise cuboid shape with a width a, a length b, and a depth c, wherein e.g. b > a and b > c, and wherein said length b e.g. corresponds to the abovementioned maximum length dimension L1, wherein preferably b < 15 mm, or more preferably b < 1.5mm. These dimensions enable a compact structure for the complete probe 100, so that efficient deployment e.g. in blood vessels and other regions of the human and/or animal body is possible, e.g., by injecting the probe 100 to the target region.

Advantageously, according to further examples, the housing 110 of the probe 100 may be designed such that its outer dimension(s) is/are not substantially larger than said maximum length dimension L1 of the element 120 having a permanent magnetic dipole moment.

Applicant believes further miniaturization of the element 120 having a permanent magnetic dipole moment and the complete probe 100 comprising said element 120 having a permanent magnetic dipole moment may be possible. For example, a maximum linear dimension for the element 120 having a permanent magnetic dipole moment of e.g. in the micrometer (pm) or even nanometer (nm) range may be possible. Such further miniaturization may enable the probe 100 according to the examples to have largest linear outer dimensions of e.g. few hundreds of micrometers or few hundreds of nanometers.

According to a further example, said element 120 having a permanent magnetic dipole moment is a permanent magnet with a remanent magnetic flux density of at least about 0.1 Tesla, T, (1 T = (1 kg)/(A^{∗}s²) ), preferably of at least about 1.4 Tesla, which can enable an external magnetic field to provide a high driving torque even for elements 120 having a permanent magnetic dipole moment with maximum linear dimensions L1 in the micrometer or nanometer range.

As an example, magnetic material of the NdFeB-type, i.e. comprising an alloy of neodymium (Nd), iron (Fe) and boron (B), e.g. Nd2Fe14B, may be used to form the element 120 having a permanent magnetic dipole moment and/or components thereof. Permanent magnets made of the above mentioned neodymium alloy may also be denoted as "supermagnets" due to their comparatively high remanent magnetic flux density.

According to some examples, the abovementioned bar magnet type having cuboid shape is particularly preferred.

However, according to further embodiments, other types of elements having a permanent magnetic dipole moment with different shapes may also be used within the probe.

Optionally, the probe 100 may comprise an electronic control unit, cf. the dashed line block 130 of Fig. 1. Said optional electronic control unit 130 may e.g. control an operation of the probe 100, e.g., by determining at least one of: a frequency and/or amplitude of the external magnetic field applied to propel the rotational movement of the element 120 having a permanent magnetic dipole moment, or a rotational speed of the element 120 having a permanent magnetic dipole moment in the probe 100. The electronic control unit 130 may e.g. comprise an ASIC (application specific integrated circuit), a microcontroller, a digital signal processor (DSP), discrete logic elements, a programmable logic circuit or any combination thereof.

According to some examples, said electronic control unit 130 may wirelessly transmit control information to an external device, e.g. causing said external device to alter parameters (amplitude and/or frequency) of said time-varying magnetic fields from the external source.

Fig. 2A depicts the probe 100 of Figures 1 according to a further embodiment 100a. While the configuration of its element 120 having a permanent magnetic dipole moment may be similar to the configuration of the probe 100 according to Fig. 1, the probe 100a of Fig. 2A comprises an induction coil 140 located so that rotation of said rotatable element 120 having a permanent magnetic dipole moment produces an electromotive force (EMF) across said induction coil 140.

As illustrated, a controllable electric resistor 142 may be connected electrically across said induction coil 140 so that the EMF produced in the induction coil 140 drives an electrical current in the resistor 142.

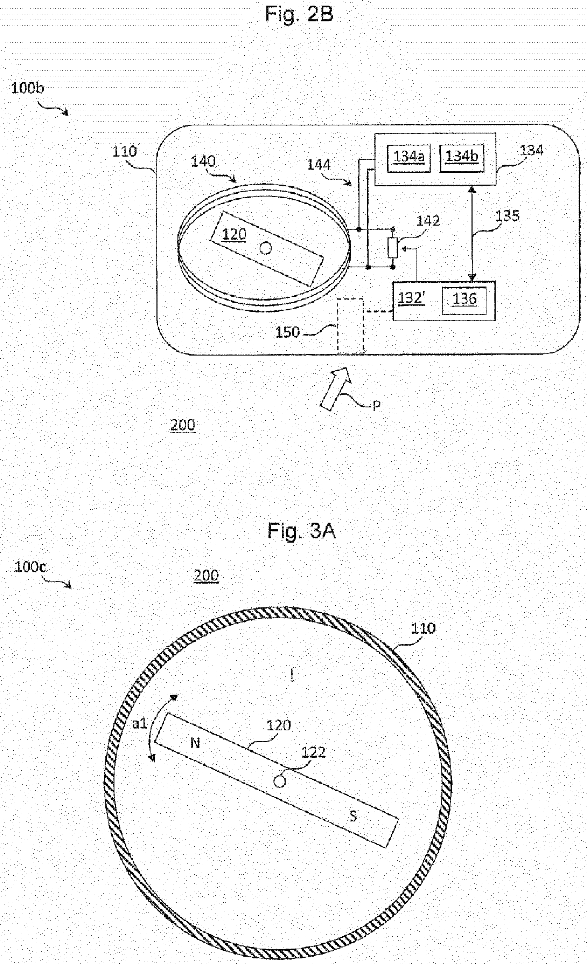

Further, the probe 100a of Fig. 2A comprises a control unit 132 that may have a structure similar to the (optional) electronic control unit 130 explained above with reference to Fig. 1. Presently, the electronic control unit 132 of Fig. 2 is configured to control the resistor 142 or another electrical power dissipator and thereby the amount of electric energy dissipated within said resistor 142. As is well known, the dissipation of said amount of electric energy causes a corresponding reduction of the rotational energy and thus, also can slow the rotational speed of the element 120 having a permanent magnetic dipole moment.

Hence, by modulating the resistance of the resistor 142, in embodiments where the resistance is controllable, the electronic control unit 132 may effect a corresponding modulation of the magnetic field generated by the rotating element 120 having a permanent magnetic dipole moment. Said modulation of the induced magnetic field may be detected by a magnetic receiver coil placed outside the probe 100a.

Thus, data transfer from the probe 100a to an external device may be enabled by a data-stream-controlled modulation of the magnetic field induced by the element 120 having a permanent magnetic dipole moment.

This modulation mechanism may enable data transfer rates of, at least, a kilobit-per-second, which may be sufficient, e.g., for transmitting measurement data from sensors (e.g., pressure, temperature and pH values) and the like in or on the exterior of the probe 100a.

According to an example, the resistor 142 may be controlled by a digital control circuit or an analog control circuit of the electronic control unit 132.

According to a further example, a value for the electric resistance of the resistor 142 may range between 0 Ohms (short circuit) and open circuit, wherein preferably any intermediate values (or at least a minimum number of different intermediate values) may also be set by means of analog or digital control. Thus, the energy extraction from the element 120 having a permanent magnetic dipole moment by the induction coil 140 may be controllable.

According to further examples, the probe 100a may comprise one or more sensors 150, which may be, e.g., partly integrated into the housing 110 of the probe 100a, and which may be configured to measure one or more physical parameters P of the probe 100a and/or a medium 200 surrounding the probe 100a, e.g., temperature, pressure, pH value, pulse rate, and the like and/or may provide image(s) of the local environment. In these embodiments, the control unit 132 may process sensor signals and/or data provided by said sensors 150. Also, the sensor signals or any data derived therefrom may be transmitted to an external device, e.g. by modulating a controllable version of the electric resistor 142 and thus, by modulating an angular speed of the element 120 having a permanent magnetic dipole moment as already explained above.

According to further examples, it is also possible to provide an analog or digital control circuit receiving sensor signal(s) from said sensor(s) 150 and directly controlling the resistor 142 depending thereon.

Fig. 2B depicts a probe 100 of Fig. 1 according to a further example 100b. While the configuration of the element 120 having a permanent magnetic dipole moment and the induction coil 140 and the resistor 142 may be similar or identical to the configuration of the probe 100a according to Fig. 2A, the probe 100b of Fig. 2B additionally comprises an energy storage unit 134 for, at least, temporarily storing electrical energy derived from the rotational movement of the element 120 having a permanent magnetic dipole moment.

For example, the energy storage unit 134 may be connected in parallel to the induction coil 140 and the resistor 142, cf. the lines 144 so that an EMF magnetically induced across the induction coil 140 can electrically drive currents in the resistor 142 and the energy storage unit 134. The energy storage unit 134 may comprise a rectifier circuit 134a for rectifying a voltage induced into the induction coil 140 and a power stabilizer circuit 134b for buffering a rectified voltage provided at an output of the rectifier circuit 134a. The power stabilizer circuit 134b may e.g. comprise a capacitor, for example a double-layer capacitor ("ultracap").

A control device 132' may be provided receiving sensor signals from optional sensors 150. Also, the control device may monitor a voltage output by the power stabilizer circuit 134b, cf. double arrow 135, and may control operation of the resistor 142 for transmitting data to an external device (not shown).

The control device 132' may further be configured to receive information transmitted by an external device, e.g. a device providing the external magnetic field for driving rotational movement of the element 120 having a permanent magnetic dipole moment. A control signal controlling said external magnetic field may be modulated (e.g., by said external device), similarly to the mechanism explained above with reference to the resistor 142 or in any other way enabling modulation of said external magnetic field, and such modulation may be detected by the control device 132'. This mechanism may be used for transmitting data and/or control commands to and/or from an external device from and/or to the control unit 132' of the probe 100b.

According to Applicant's analysis, such one-direction or two-direction communication of data and/or control instructions have proceeded at rates, e.g., in the range of, at least, several kilobits per seconds.

Figure 3A schematically depicts a probe 100c according to a further example. The probe 100c has a substantially spherical shape, and an element 120 having a permanent magnetic dipole moment is rotatably arranged (cf. double arrow a1) within a housing 110. Presently, the element 120 having a permanent magnetic dipole moment may have the form of a bar magnet type, e.g. having substantially cuboid or parallelepiped shape. Exemplarily the magnetic north and south poles of the element 120 having a permanent magnetic dipole moment are also indicated in Fig. 3A.

Alternatively to the basically spherical shape of Fig. 3A, an ellipsoidal shape or further shapes are also possible for the housing 110.

Presently, the element 120 having a permanent magnetic dipole moment comprises an inner bearing implemented by means of a shaft arranged at the position of the axis of rotation 122, wherein said shaft is mounted to an interior I of the housing 110.

In contrast, Fig. 3B depicts a further example 100d of the probe 100 of Fig. 1, wherein said element 120 having a permanent magnetic dipole moment comprises an external bearing. This is enabled by providing axial end sections 120a, 120b of the element 120 having a permanent magnetic dipole moment with suitable gliding properties and/or rounded edges so that said axial end sections 120a, 120b may be guided in a sliding movement by the inner surface 110a of the housing 110. In effect, the element 120 having a permanent magnetic dipole moment is also rotatably arranged (cf. double arrow a2) around a (virtual) axis of rotation 122a. For facilitating support of the gliding movement of the axial end sections 120a, 120b of the element 120 having a permanent magnetic dipole moment on the inner surface 110a of the housing, said inner surface 110a may comprise a suitable material and/or coating or the like which exhibits sufficient gliding properties with the axial end sections of the element having a permanent magnetic dipole moment. Alternatively or additionally, the element 120 having a permanent magnetic dipole moment or at least its axial end sections 120a, 120b may be coated with a material having good gliding properties with respect to the inner surface 110a.

Fig. 3C depicts yet another example 100e of the probe 100 of Fig. 1, wherein an annular groove 110b is provided within the inner surface 110a of the housing, said annular groove 110b guiding the axial end sections 120a, 120b of the element 120 having a permanent magnetic dipole moment, thus again constituting an external bearing for said element 120 having a permanent magnetic dipole moment.

As a rotational movement of the element 120 having a permanent magnetic dipole moment basically occupies a circular disc shaped volume portion of the basically spherical interior I of the housing 110, for example basically centered around the drawing plane of Fig. 3C, other volume regions of the interior I of the housing 110, e.g. further away from the drawing plane of Fig. 3C, may be used for integrating components such as induction coil(s) 140, control unit(s) 130, 132, 132' and the like.

According to an example, one or more induction coil(s) may also be placed on the inner surface (Fig. 3A) of the housing 110.

According to a preferred example, the element 120 having a permanent magnetic dipole moment is preferably formed such that it comprises a mass distribution symmetrical to its axis of rotation, to avoid vibrations and energy losses caused by undesired vibrations.

However, according to other examples, it may be desired to provide the element 120 having a permanent magnetic dipole moment with an asymmetric mass distribution with respect to its axis of rotation to enable vibrations of said element 120 having a permanent magnetic dipole moment and the probe 100e containing it to be generated by driving rotational movement of the element 120 having a permanent magnetic dipole moment. This enables to introduce structure-borne noise (ranging from low frequencies of a few Hz up to ultrasonic frequency ranges of several tens of kHz) into an area surrounding the probe 100e, e.g. for effecting movement or even local heating of a surrounding medium.

According to an example, the housing 110 is hermetically sealed, so that no fluids (liquids and/or gases) surrounding the probe 100e may enter the interior I.

According to a further embodiment, the interior I may comprise a vacuum or at least a partial vacuum, which ensures substantially unimpeded rotational movement of the element 120 having a permanent magnetic dipole moment.

According to further examples, the interior I of the probe 100e may also comprise a gas and/or a liquid, e.g. for supporting the element having a permanent magnetic dipole moment. In these embodiments, especially with microscale dimensions (i.e., the element 120 having a permanent magnetic dipole moment having a maximum length dimension between few µm and few thousands of pm) or nanoscale dimensions (i.e., the element 120 having a permanent magnetic dipole moment having a maximum length dimension between few nm and few thousands of nm) it is preferred to provide a gas or liquid within said interior I which comprises a sufficiently low viscosity in order not to impede rotational movement of the element having a permanent magnetic dipole moment. However, for microscale or nanoscale elements having a permanent magnetic dipole moment, a partial vacuum or a total vacuum may also be contemplated.

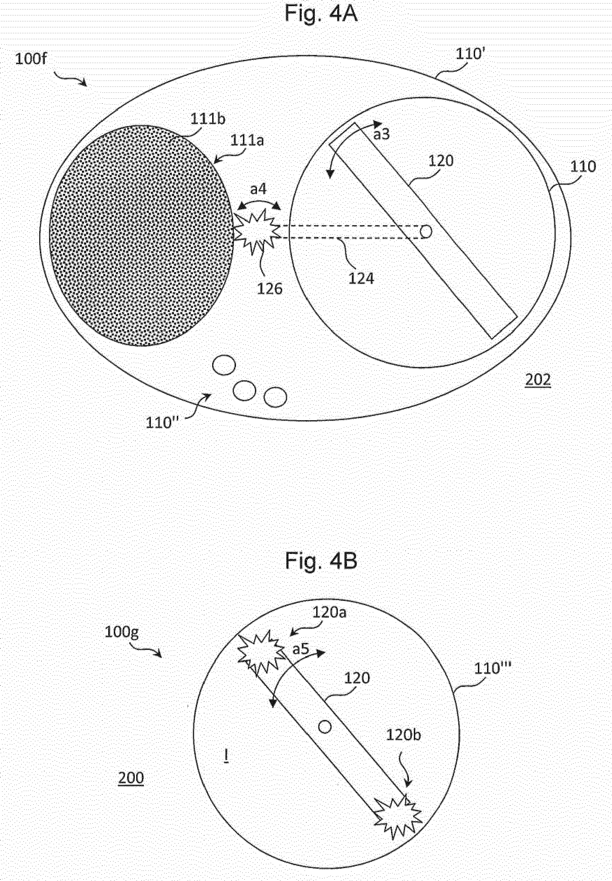

Figure 4A schematically depicts a probe 100f according to a further embodiment of the probe 100 of Fig. 1. The probe 100f comprises an element 120 having a permanent magnetic dipole moment rotatably arranged in a housing 110, cf. double arrow a3. The housing 110 is arranged in a capsule 110', which also comprises a container 111a comprising an agent 111b such as a drug which is to be released from the capsule 110' inside a human body.

Presently, the element 120 having a permanent magnetic dipole moment is coupled to a first axial end section of a shaft 124, which defines an axis of rotation of the element 120 having a permanent magnetic dipole moment. A second axial end section of the shaft 124 protrudes from the housing 110 and comprises a drilling tip 126 which is coupled to the shaft 124 in a torque-proof manner (cf. double arrow a4 indicating rotation of the drilling tip 126) so that the drilling tip 126 will open the container 111a once the element 120 having a permanent magnetic dipole moment drives the shaft 124. Thus, the agent 111b comprised in the container 111a may be set free within the capsule 110' in a controlled manner, i.e. by activating rotational movement of the element 120 having a permanent magnetic dipole moment by application of a corresponding external magnetic field.

Preferably, said capsule 110' may comprise one or more openings 110" so that the agent - once set free from its container 111a - may leave the capsule 110' and reach a target area 202 surrounding the capsule 110'.

Alternatively, the wall material of the capsule 110' may be configured such that human or animal tissue may substantially not enter the interior of the capsule, but such that an agent carried within container 111a may be released through said wall.

Figure 4B schematically depicts a probe 100g according to a further example of the probe 100 of Fig. 1. The element 120 having a permanent magnetic dipole moment carries at its axial end sections blades or other structures suitable of severing a material of the housing 110"' once the element 120 having a permanent magnetic dipole moment is rotating, cf. arrow a5. Thus, an agent such as a drug (not shown) arranged in the interior I of the probe 100g may be controllably set free by activation of rotation of the element 120 having a permanent magnetic dipole moment once the probe 100g is positioned within a target region 200 e.g. within a human body.

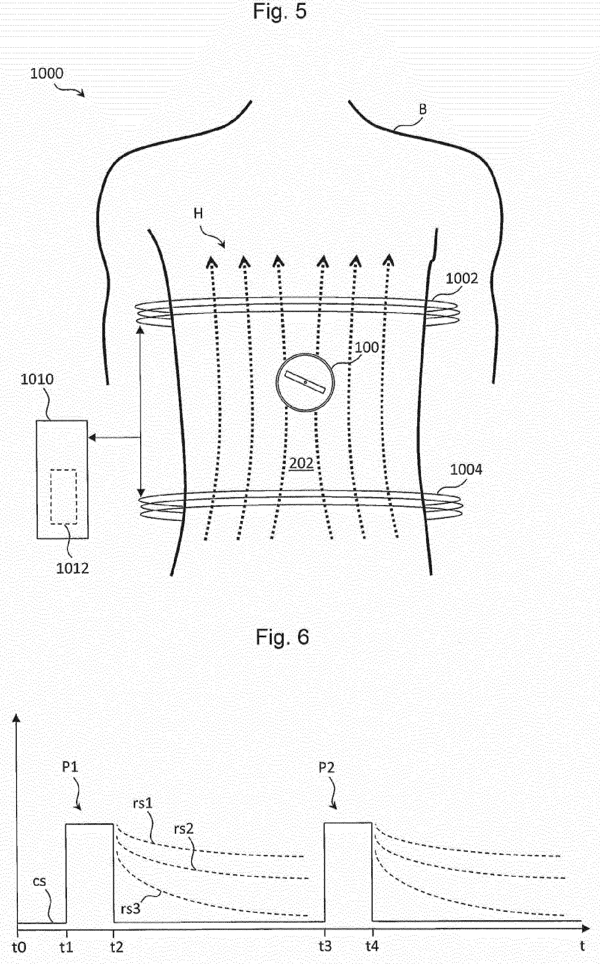

Figure 5 schematically depicts an operational scenario of a system 1000 that uses any embodiment of the probe 100 of Fig. 1 in a human or animal body B. For example, the probe 100 is illustrated as being positioned within a gastrointestinal (GI) tract 202 of the body B. Such position may e.g. be attained by oral insertion and travel of the probe 100 to the illustrated position in the GI tract, or by injection, or by locally inserting the probe 100 by using another (e.g., conventional) probe for analyzing a human body's GI tract.

The probe 100 may e.g. comprise any of the examples 100a, 100b, 100c, 100d, 100e, 100f, and 100g as explained above with reference to the Figures 1 to 4B or any combination thereof.

The system 1000 comprises a magnetic field generator having a control unit 1010 and a pair of Helmholtz coils 1002, 1004 arranged outside and around the GI region of the human or animal body B, e.g., completely outside of the body B.

The Helmholtz coils 1002, 1004 may easily be put in place around the human or animal body B e.g. by sliding them axially over the body. Alternatively or in addition, one or more coils connected to said control unit 1010 could also be placed in a piece of clothing such as e.g. a vest, which may be put on by a patient prior to operating the system 1000.

The Helmholtz coils 1002, 1004 may e.g. be used to provide a basically homogenous static or time variable magnetic field H therebetween in a per se known manner. A control current for this may be provided by the control unit 1010.

The application of the magnetic field H will cause the magnetic dipole moment of the element 120 having a permanent magnetic dipole moment (Fig. 1) of the probe 100 to significantly or substantially align with the field lines (static case) or to rotate (dynamic case), e.g. depending on a frequency of the alternating magnetic field H.

In other words, the element 120 having a permanent magnetic dipole moment, which may preferably be a "supermagnet" (i.e., with high remanent magnetic flux density), interacts with the externally generated magnetic field H, whereby a type of "synchronous motor" may be formed, wherein the element 120 having a permanent magnetic dipole moment corresponds to the rotor of the "synchronous motor" and wherein the Helmholtz coils 1002, 1004 correspond to the stator of the "synchronous motor". Advantageously, employing the principle of the various embodiments enables to efficiently transfer energy from outside the probe 100, even from outside the human or animal body B, to the probe 100, which is placed inside the body B, without requiring any electrical connections to the body. Also, while the "stator" (i.e., the coils 1002, 1004) may comprise "macro-dimensions", it is possible to design the "rotor", namely the element 120 having a permanent magnetic dipole moment, as a micro- or even nanoscale component thus offering increased operational flexibility such as drug delivery or other probe applications directly within smaller blood vessels or other parts of the human or animal body B where conventional probes cannot be used due to their size.

Further advantageously, an energy flow to the probe 100 may be controlled from the outside, thus externally triggering effects for in-body actuation and even signal transmission into and out of the body are possible as already discussed.

According to an example, the control unit 1010 may be configured to provide an alternating magnetic field for driving rotational movement of the element 120 having a permanent magnetic dipole moment of the probe 100 (Fig. 5) inside the body B.

According to further examples, the control unit 1010 may be configured to provide a rotating magnetic field. For this purpose, according to further embodiments, it is also possible to provide more than two coils 1002, 1004 for generating the magnetic field H. For example, by providing two further Helmholtz coils arranged orthogonal with respect to the two Helmholtz coils 1002, 1004 as depicted by Fig. 5, rotating magnetic fields can be generated.

According to an example, a rotational speed of the element 120 having a permanent magnetic dipole moment is determined by a frequency of a periodic field change of the magnetic field H generated by the coils 1002, 1004.

According to a further example, by providing a static magnetic field H, the element 120 having a permanent magnetic dipole moment (Fig. 1) of the probe 100 may be aligned with said static magnetic field H, which may e.g. be used for moving the element 120 having a permanent magnetic dipole moment in a defined position with respect to the coils 1002, 1004. After that, by inverting the magnetic field or by applying a periodically alternating magnetic field, rotational movement of the element 120 having a permanent magnetic dipole moment may be produced.

In the following, an example calculation of the drive torque of the element 120 having a permanent magnetic dipole moment is presented for an exemplary configuration of the probe as follows.

According to the example, it is assumed that the element 120 having a permanent magnetic dipole moment has basically cuboid shape with a length L1 of about 5 mm, and a width and depth of each about 1 mm. The volume V of the element 120 having a permanent magnetic dipole moment is thus about 5 x 10⁻⁹ m³. The magnetic remanence flux density Br is assumed to equal about 1,47 T (Tesla), which may e.g. be attained by using magnet material of the NdFeB "N50H" type. With the permeability of free space of about µ0=1,26 x 10⁻⁶ Vs / Am, a magnetic dipole moment m of the element 120 having a permanent magnetic dipole moment is obtained as follows: m = Br x V / µ0 = 5,8 x 10⁻³ Am².

Further as an example, it is assumed that the magnetic induction of the magnetic field H generated by the Helmholtz coils 1002, 1004 at the position of the element 120 having a permanent magnetic dipole is: B = 10 mT. Hence, the magnetic torque T obtained at the element 120 having a permanent magnetic dipole moment is: T = m x B (vector product of magnetic dipole moment m and magnetic induction B), T = 5,8 x 10⁻⁵ Nm.

As a result, the driving force on the element 120 having a permanent magnetic dipole moment caused by the externally applied magnetic field H is F = T / L1 = 1,2 x 10⁻² N.

According to a further embodiment, the magnetic field generator 1002, 1004, 1010 exemplarily depicted by Fig. 5 is configured to, in a first operational state, provide a temporally alternating magnetic field, particularly to drive a rotational movement of the element 120 having a permanent magnetic dipole moment of said at least one probe 100, wherein optionally, said magnetic field generator 1002, 1004, 1010 is configured to, in a second operational state, provide a substantially static magnetic field, particularly to align the element 120 having a permanent magnetic dipole moment of said at least one probe 100 in a predetermined direction depending on said substantially static magnetic field.

For example, the first operational state may be employed to supply the probe 100 with mechanical energy, while the second operational state may be used to align the element 120 having a permanent magnetic dipole moment with the magnetic field provided in said second operational state, e.g. to establish a defined starting position prior to entering the first operational mode.

According to a further example, said system 1000 comprises an optional receiver 1012 configured to receive an alternating magnetic signal from the element 120 having a permanent magnetic dipole moment of said probe 100. Said receiver 1012 may e.g. comprise a distinct magnetic coil capable of detecting a time variable magnetic flux generated by the rotating element having a permanent magnetic dipole moment of the probe 100.

Alternatively, one or more of the coils 1002, 1004 of the magnetic field generator 1002, 1004, 1010 may also be used for detecting a time variable magnetic flux generated by the rotating element having a permanent magnetic dipole moment of the probe 100, e.g. in a time division duplexed manner with respect to generating a magnetic field for driving rotational movement of the element having a permanent magnetic dipole moment. By influencing the rotational movement of the element 120 having a permanent magnetic dipole moment, the probe 100 may transmit information to the receiver 1012. For example, the embodiments 100a, 100b of Fig. 2A, 2B may influence the rotational movement of their element 120 having a permanent magnetic dipole moment by means of the electromagnetically coupled induction coil 140 and the controllable resistor 142, as already explained above.

Figure 6 schematically depicts an example of a time diagram of operational parameters of the system 1000 according to Figure 5.

Reference sign t0 indicates a starting time at which the probe 100 is deployed in the GI tract of the body B (Fig. 5) and is ready for operation. A control signal cs indicates by means of two pulses P1, P2 two time intervals (t1, t2), (t3, t4) during which an alternating magnetic field H (Fig. 5) is generating by the magnetic field generator 1002, 1004, 1010. More specifically, this is achieved by applying corresponding alternating currents to both Helmholtz coils 1002, 1004 using the control unit 1010. During the further time intervals (t0, t1), (t2, t3) and after time t4, no magnetic field is generated.

As already explained above, the alternating magnetic field H applied during e.g. the time interval (t1, t2) causes a rotational movement of the element 120 having a permanent magnetic dipole moment of the probe 100 (Fig. 5), wherein a rotational speed depends on the frequency of the alternating magnetic field H. After deactivation of said alternating magnetic field H, e.g. for t > t2, the rotational speed of the element 120 having a permanent magnetic dipole moment will decrease, primarily due to friction losses. After the second pulse P2, this scenario repeats.

However, a controllable damping, which may be attained by the induction coil 140 and the damping resistor 142, cf. e.g. the configuration of the probe depicted by Fig. 2A, may also influence the decrease of the rotational speed of the element 120 having a permanent magnetic dipole moment after the pulse(s) P1, P2. Fig. 6 depicts three curves rs1, rs2, rs3 for potential variations in time of the rotational speed of the elements having a permanent magnetic dipole moment caused by different damping actions by means of the elements 140, 142. Advantageously, these different variations in time may be detected by the receiver 1012, which may e.g. measure an induction voltage induced by the magnetic field of the rotating element 120 having a permanent magnetic dipole moment in a receiver coil associated with the receiver 1012. As an example, as for the time interval (t2, t3), no magnetic field generation for driving the element 120 having a permanent magnetic dipole moment is required, one or more of the Helmholtz coils 1002, 1004 could be used by the receiver 1012 as receiver coils for measuring the induction voltage induced by the magnetic field of the rotating element 120 having a permanent magnetic dipole moment.

This way, it is possible to transmit data from the probe 100 to an external device 1012, so that a bidirectional communication between the probe and an external device may be attained at least for some of the embodiments explained above.

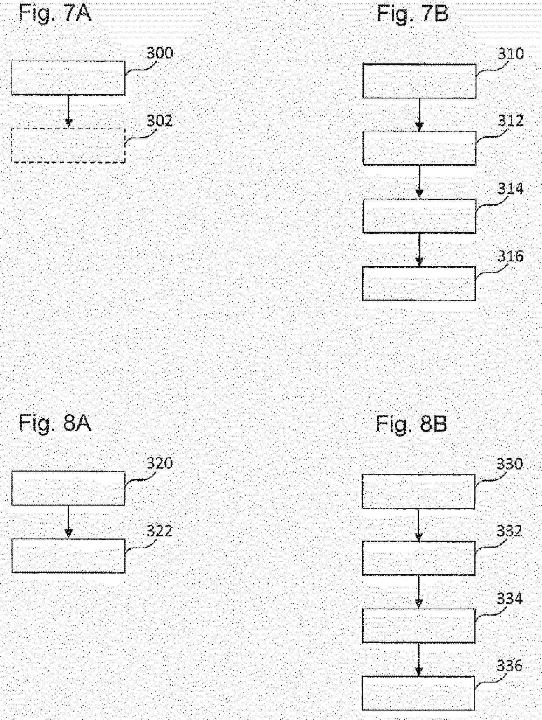

Figure 7A schematically depicts a simplified flow-chart of a method of operating a probe according to the discussed examples.

In step 300, the probe receives energy from an external magnetic field, e.g. field H as depicted by Fig. 5. A drive torque resulting from the external magnetic field in combination with the non-vanishing magnetic dipole moment of the element 120 having a permanent magnetic dipole moment (Fig. 1) drives rotational movement of said element 120 having a permanent magnetic dipole moment, cf. double arrow a1 of Fig. 1. In other words, at least a portion of the energy received from the external magnetic field H is transformed to mechanical, e.g. rotational, energy of the element 120 having a permanent magnetic dipole moment within the probe according to the embodiments. After that, optionally, in step 302 (Fig. 7A), at least a portion of the so received energy may be used for a specific action such as conversion to electric energy and/or thermal energy or sound waves, e.g. ultrasonic waves, or for driving an actuator of which the probe 100 is equipped.

Figure 7B schematically depicts a simplified flow-chart of a method of operating a probe 100b (Fig. 2B) according to a further example. In step 310, the probe 100b receives energy from the externally generated magnetic field H (Fig. 5). Also in this step 310, at least a portion of said received energy is transformed into electric energy and temporarily stored or buffered by means of the energy storage unit 134. In step 312, the probe 100b receives a control command from the control unit 1010, wherein said control command is e.g. provided by modulating the alternating magnetic field in a way than can be detected and demodulated by the local control device 132' of the probe 100b. Said control command may e.g. instruct the probe 100b or its local control device 132', respectively, to evaluate a signal of a sensor 150 comprised within the probe 100b in order to obtain a measurement value, e.g. a pressure value of a medium surrounding the probe 100b. This is done in subsequent step 314 of Fig. 7B. After said evaluation, the measured pressure value is transmitted from the probe 100b to the external device 1010 or its receiver 1032, respectively, in step 316 (Fig. 7B) by modulating an inductive damping of the rotating element 120 having a permanent magnetic dipole moment (Fig. 2B) by means of the resistor 142, as explained above with reference to Fig. 6.

According to further examples, the probe may also comprise a, preferably non-volatile, memory unit 136 for storing measurement values obtained from the sensor(s) 150 (Fig. 2B), wherein said memory unit may e.g. be included in a control device 132'.

Figure 8A schematically depicts a simplified flow-chart of a method of operating a system 1000 (Fig. 5) according the embodiments. In step 320, the magnetic field generator 1002, 1004, 1010 (Fig. 5) of the system 1000 assumes a second operational state in which a substantially static magnetic field H is generated to align the element 120 having a permanent magnetic dipole moment (Fig. 1) of the probe 100 in a predetermined direction with respect to said static magnetic field H. Subsequently, in step 322, the magnetic field generator 1002, 1004, 1010 (Fig. 5) of the system 1000 assumes a first operational state in which a temporally alternating magnetic field H is generated to drive a rotational movement of the element 120 having a permanent magnetic dipole moment of the probe, which may e.g. be used for continued energy supply of the probe 100.

While according to further embodiments, the step 320 of aligning may be omitted, according to other embodiments it may be particularly beneficial as it improves an efficient initialization of rotational movement within the first operating state 322.

Figure 8B schematically depicts a simplified flow-chart of a method of operating a system 1000 (Fig. 5) according to a further example. In the present case, the probe may comprise elements of the embodiment of Fig. 2B and of the embodiment of Fig. 4A. In step 330, the magnetic field generator 1002, 1004, 1010 (Fig. 5) of the system 1000 assumes its first operational state in which a temporally alternating magnetic field H is generated to drive a rotational movement of the element 120 having a permanent magnetic dipole moment of the probe. In step 332, the control unit 1010 transmits a control command to the probe instructing it to start a measurement, e.g. using a sensor 150. After the measurement, the probe transmits the measured value to the control unit 1010, which may also be performed in step 332. In subsequent step 334, the control unit 1010 receives the measured value by means of its receiver 1012. After that, in step 336, the control unit 1010 transmits a further control command to the probe instructing it to release a drug into the surroundings of the probe. This may e.g. be attained using the techniques explained above with respect to Fig. 4A, 4B.

In the following paragraphs, further examples and advantages are presented.

The probe and the system according to the examples may advantageously be used in the fields of disease management, remote doctor control, personal wellness, but they are not limited to applications in the human or animal body.

Advantageously, sensors and/or actuators may be provided for use within the human and/or animal body (or in any other target system) in combination with the probe according to the embodiments, which enables a direct energy supply of said sensors and/or actuators from the outside, by means of the system 1000 according to the examples.

Also, if such sensors and/or actuators need to communicate with and require control from instances outside the body to allow monitoring and maintaining/regaining human and/or animal health and wellness, this may be attained by combining them with the probe according to the examples and by using a system 1000 according to the examples. In other words, according to some examples, the probe may be equipped with (conventional) sensors and/or actuators and provides them with the power required for operating within the human and/or animal body, without requiring direct electric connections to the probe and/or to the sensors and/or actuators themselves.

According to one aspect, the probe may be implantable and/or injectable and/or ingestible.

The probe according to the examples may e.g. be designed as a nanoscale, microscale or larger device, as it is possible to manufacture elements 120 having a permanent magnetic dipole moment according to the embodiments with nanoscale, microscale, or larger linear dimension(s), because particularly NdFeB-based magnets have a fine-crystalline structure. A fabrication process for elements 120 having a permanent magnetic dipole moment may comprise steps of milling the raw materials, compression of the so obtained powder, and finally sintering of the magnetic material. On the other hand, in view of the high remanent magnetic flux density that can be attained with NdFeB magnets, even with nano- or microscale elements 120 having a permanent magnetic dipole moment of this type significant torque values may be attained, which enables an efficient energy transfer from outside the probe to the element 120 having a permanent magnetic dipole moment rotatably arranged in the housing of the probe.

In comparison, this would not typically be easily achieved with conventional magnetic coils, as with a magnetic coil of 100 turns, a length of 10 mm and a diameter of 4 mm, a resulting magnetic induction of only about 1.2 x 10⁻⁴ T could be attained with an electric current of about 100 mA flowing through the coil. Evidently, the elements having a permanent magnetic dipole moment according to the embodiments are superior for in-body use as they enable to provide nano- or microscale elements 120 having a permanent magnetic dipole moment enabling efficient energy transfer from outside the body into a probe positioned within the body.

Still further, according to Applicant's analysis, the rotation of such elements having magnetic dipole moments with linear dimensions in the micrometer-range and larger in a fluid can be performed in a controlled way by applying suitable external magnetic fields. According to some embodiments, said external magnetic fields may e.g. comprise magnetic flux densities in the range of about few µT (microtesla) up to about few mT (millitesla).

Advantageously, the principle according to the examples may be used for targeted in-body drug delivery, gaining diagnostics information, and also for taking biopsy samples, as it enables energy supply of and information exchange with nano-/microscale probes inside the body.

Further advantageously, the principle according to the examples enables an efficient data transfer between an external device 1010 (Fig. 5) and the probe 100, even if the probe 100 is positioned deeply within body tissue.

These advantages cannot be attained with conventional approaches based on RF signal transmission, due to reduced penetration depths of such RF signals. Still further, the principle according to the examples advantageously enables to directly use mechanical energy, e.g. obtained at a shaft 124 (Fig. 4A) driven by the element 120 having a permanent magnetic dipole moment, thus avoiding conversion losses which would occur with transformation to other forms of energy. Also, in-body battery use may be avoided according to some examples.

As for most forms of in-body actuation, mechanical energy is required, e.g. for drug release, taking biopsy samples (e.g. by using a small mechanically driven razor), or provision of device movement (by e.g. propelling elements), the principle according to the embodiments is particularly beneficial because it provides for a direct supply with mechanical energy within the probe 100, even if such device comprises micro- or nanoscale dimensions.

The description and drawings merely illustrate the principles of the inventions. The invention is defined by appended claims 1-12.

## Claims

1. An apparatus for use in a human or animal body, comprising a probe (100; 100a; 100b; 100c; 100d; 100e; 100f; 100g), the probe comprising a housing (110), at least one element (120) having a permanent magnetic dipole moment being rotatable within said housing (110), and an electrical circuit (C), said circuit (C) comprising at least one induction coil (140) that is magnetically coupled with the element (120) having a permanent magnetic dipole moment and a controllable electric resistor (142) connected in parallel with said induction coil (140), the element having a permanent magnetic dipole moment being configured to generate an electromotive force in said at least one induction coil (140) in response to being rotated, wherein said probe is configured to wirelessly transmit data to an external device by modulating a resistance of the resistor (142).

2. The apparatus according to claim 1, wherein said element (120) having a permanent magnetic dipole moment has a maximum length dimension (L1) smaller than about 15 millimeter.

3. The apparatus according to one of the preceding claims, wherein said element (120) comprises a remanent magnetic flux density of at least about 0.1 Tesla.

4. The apparatus according to one of the preceding claims, further comprising an electrical control unit (130; 132; 132') in said housing (110).

5. The apparatus according to one of the preceding claims, wherein said housing (110) has a basically ellipsoidal or spherical shape.

6. The apparatus according to one of the preceding claims, wherein said probe (100; 100a; 100b; 100c; 100d; 100e; 100f; 100g) comprises a device for transforming mechanical rotational energy of said element (120) having a permanent magnetic dipole moment into a flow of a fluid surrounding the housing (110) of said probe, and/or for transforming mechanical rotational energy of said element (120) having a permanent magnetic dipole moment into movement of said probe, and/or for transforming said mechanical rotation energy of said element (120) having a permanent magnetic dipole moment into acoustic energy.

7. The apparatus according to one of the preceding claims, wherein said probe (100; 100a; 100b; 100c; 100d; 100e; 100f; 100g) is configured to carry at least a drug releasable therefrom into the human or animal body.

8. Method of operating the apparatus of claim 1, wherein said probe wirelessly transmits data to said external device by modulating a resistance of the resistor (142).

9. Method according to claim 8, wherein said probe (100; 100a; 100b; 100c; 100d; 100e; 100f; 100g) transforms mechanical rotation energy of said element (120) having a permanent magnetic dipole moment into at least one of: a) electrical energy, b) thermal energy, c) a flow of a fluid surrounding the housing (110) of said probe (100; 100a; 100b; 100c; 100d; 100e; 100f; 100g) and/or within the housing (110) of said probe (100; 100a; 100b; 100c; 100d; 100e; 100f; 100g), d) propulsion energy for moving said probe (100; 100a; 100b; 100c; 100d; 100e; 100f; 100g), e) acoustic energy.

10. System (1000) comprising an apparatus with at least one probe (100; 100a; 100b; 100c; 100d; 100e; 100f; 100g) according to one of the claims 1 to 7 and a magnetic field generator (1002, 1004, 1010) configured to provide a temporally varying magnetic field (H) in said at least one probe (100; 100a; 100b; 100c; 100d; 100e; 100f; 100g) .

11. System (1000) according to claim 10, wherein said magnetic field generator (1002, 1004, 1010) is configured to provide a substantially static magnetic field (H) to align the element (120) having a permanent magnetic dipole moment in a selected direction.

12. System (1000) according to one of the claims 10 to 11, wherein said system (1000) comprises a receiver (1012) configured to receive a data stream from the element (120) .

## Patentansprüche

1. Vorrichtung zur Verwendung in einem menschlichen oder tierischen Körper, umfassend eine Sonde (100; 100a; 100b; 100c; 100d; 100e; 100f; 100g), wobei die Sonde ein Gehäuse (110), mindestens ein Element (120) mit einem permanentmagnetischen Dipolmoment, das innerhalb des Gehäuses (110) drehbar ist, und eine elektrische Schaltung (C) umfasst, wobei die Schaltung (C) mindestens eine Induktionsspule (140), die magnetisch mit dem Element (120) mit einem permanentmagnetischen Dipolmoment gekoppelt ist, und einen steuerbaren elektrischen Widerstand (142) umfasst, der parallel zur Induktionsspule (140) geschaltet ist, wobei das Element mit einem permanentmagnetischen Dipolmoment dazu eingerichtet ist, eine elektromotorische Kraft in der mindestens einen Induktionsspule (140) als Reaktion auf eine Drehung zu erzeugen, wobei die Sonde dazu eingerichtet ist, Daten drahtlos durch Modulieren eines Widerstandswertes des Widerstandes (142) an eine externe Einrichtung zu übertragen.

2. Vorrichtung nach Anspruch 1, wobei das Element (120) mit einem permanentmagnetischen Dipolmoment eine maximale Längenabmessung (L1) von weniger als etwa 15 Millimeter aufweist.

3. Vorrichtung nach einem der vorhergehenden Ansprüche, wobei das Element (120) eine remanente magnetische Flussdichte von mindestens etwa 0,1 Tesla aufweist.

4. Vorrichtung nach einem der vorhergehenden Ansprüche, ferner umfassend eine elektrische Steuereinheit (130; 132; 132') in dem Gehäuse (110).

5. Vorrichtung nach einem der vorhergehenden Ansprüche, wobei das Gehäuse (110) eine im Wesentlichen ellipsoidische oder kugelförmige Form aufweist.

6. Vorrichtung nach einem der vorhergehenden Ansprüche, wobei die Sonde (100; 100a; 100b; 100c; 100d; 100e; 100f; 100g) eine Einrichtung zum Umwandeln mechanischer Rotationsenergie des Elements (120) mit einem permanentmagnetischen Dipolmoment in einen das Gehäuse (110) der Sonde umgebenden Fluss einer Flüssigkeit und/oder zum Umwandeln mechanischer Rotationsenergie des Elements (120) mit einem permanentmagnetischen Dipolmoment in Bewegung der Sonde und/oder zum Umwandeln der mechanischen Rotationsenergie des Elements (120) mit einem permanentmagnetischen Dipolmoment in akustische Energie aufweist.

7. Vorrichtung nach einem der vorhergehenden Ansprüche, wobei die Sonde (100; 100a; 100b; 100c; 100d; 100e; 100f; 100g) dazu eingerichtet ist, mindestens ein daraus freisetzbares Medikament in den menschlichen oder tierischen Körper zu bringen.

8. Verfahren zum Betreiben der Vorrichtung nach Anspruch 1, wobei die Sonde drahtlos Daten an die externe Einrichtung durch Modulieren eines Widerstandswertes des Widerstandes (142) überträgt.

9. Verfahren nach Anspruch 8, wobei die Sonde (100; 100a; 100b; 100c; 100d; 100e; 100f; 100g) mechanische Rotationsenergie des Elements (120) mit einem permanentmagnetischen Dipolmoment in mindestens eines von Folgendem umwandelt: a) elektrische Energie, b) thermische Energie, c) Fluss einer das Gehäuse (110) der Sonde (100; 100a; 100b; 100c; 100d; 100e; 100f; 100g) und/oder innerhalb des Gehäuses (110) der Sonde (100; 100a; 100b; 100c; 100d; 100e; 100f; 100g) umgebenden Flüssigkeit, d) Vortriebsenergie zum Bewegen der Sonde (100; 100a; 100b; 100c; 100d; 100e; 100f; 100g), e) akustische Energie.

10. System (1000), umfassend eine Vorrichtung mit mindestens einer Sonde (100; 100a; 100b; 100c; 100d; 100e; 100f; 100g) nach einem der Ansprüche 1 bis 7 und einen Magnetfeldgenerator (1002, 1004, 1010), der dazu eingerichtet ist, ein zeitlich veränderliches Magnetfeld (H) in der mindestens einen Sonde (100; 100a; 100b; 100c; 100d; 100e; 100f; 100g) zu erzeugen.

11. System (1000) nach Anspruch 10, wobei der Magnetfeldgenerator (1002, 1004, 1010) dazu eingerichtet ist, ein im Wesentlichen statisches Magnetfeld (H) zu liefern, um das Element (120) mit einem permanentmagnetischen Dipolmoment in einer ausgewählten Richtung auszurichten.

12. System (1000) nach einem der Ansprüche 10 bis 11, wobei das System (1000) einen Empfänger (1012) umfasst, der dazu eingerichtet ist, einen Datenstrom von dem Element (120) zu empfangen.

## Revendications

1. Appareil destiné à être utilisé dans un corps humain ou animal, comprenant une sonde (100 ; 100a ; 100b ; 100c ; 100d ; 100e ; 100f ; 100g), la sonde comprenant un logement (110), au moins un élément (120) ayant un moment de dipôle magnétique permanent pouvant tourner à l'intérieur dudit logement (110), et un circuit électrique (C), ledit circuit (C) comprenant au moins une bobine d'induction (140) qui est couplée magnétiquement à l'élément (120) ayant un moment de dipôle magnétique permanent et une résistance électrique pouvant être commandée (142) connectée en parallèle avec ladite bobine d'induction (140), l'élément ayant un moment de dipôle magnétique permanent étant conçu pour générer une force électromotrice dans ladite au moins une bobine d'induction (140) en réponse à la mise en rotation, ladite sonde étant conçue pour transmettre sans fil des données vers un dispositif externe en modulant une résistance de la résistance (142).

2. Appareil selon la revendication 1, dans lequel ledit élément (120) ayant un moment de dipôle magnétique permanent a une longueur maximale (L1) inférieure à environ 15 millimètres.

3. Appareil selon l'une des revendications précédentes, dans lequel ledit élément (120) comprend une densité de flux magnétique rémanent d'au moins environ 0,1 tesla.

4. Appareil selon l'une des revendications précédentes, comprenant en outre une unité de commande électrique (130 ; 132 ; 132') dans ledit logement (110).

5. Appareil selon l'une des revendications précédentes, dans lequel ledit logement (110) a une forme basiquement ellipsoïdale ou sphérique.

6. Appareil selon l'une des revendications précédentes, dans lequel ladite sonde (100 ; 100a ; 100b ; 100c ; 100d ; 100e ; 100f ; 100g) comprend un dispositif pour transformer une énergie de rotation mécanique dudit élément (120) ayant un moment de dipôle magnétique permanent en un flux d'un fluide entourant le logement (110) de ladite sonde, et/ou pour transformer une énergie de rotation mécanique dudit élément (120) ayant un moment de dipôle magnétique permanent en un mouvement de ladite sonde, et/ou pour transformer ladite énergie de rotation mécanique dudit élément (120) ayant un moment de dipôle magnétique permanent en énergie acoustique.

7. Appareil selon l'une des revendications précédentes, dans lequel ladite sonde (100 ; 100a ; 100b ; 100c ; 100d ; 100e ; 100f ; 100g) est conçue pour transporter au moins un médicament pouvant être libéré depuis celle-ci dans le corps humain ou animal.

8. Procédé de fonctionnement de l'appareil selon la revendication 1, dans lequel ladite sonde transmet sans fil des données audit dispositif externe en modulant une résistance de la résistance (142).

9. Procédé selon la revendication 8, dans lequel ladite sonde (100 ; 100a ; 100b ; 100c ; 100d ; 100e ; 100f ; 100g) transforme une énergie de rotation mécanique dudit élément (120) ayant un moment de dipôle magnétique permanent en au moins une parmi : a) une énergie électrique, b) une énergie thermique, c) un flux d'un fluide entourant le logement (110) de ladite sonde (100 ; 100a ; 100b ; 100c ; 100d ; 100e ; 100f ; 100g) et/ou à l'intérieur du logement (110) de ladite sonde (100 ; 100a ; 100b ; 100c ; 100d ; 100e ; 100f ; 100g), d) une énergie de propulsion pour déplacer ladite sonde (100 ; 100a ; 100b ; 100c ; 100d ; 100e ; 100f ; 100g), e) une énergie acoustique.

10. Système (1000) comprenant un appareil équipé d'au moins une sonde (100 ; 100a ; 100b ; 100c ; 100d ; 100e ; 100f ; 100g) selon l'une des revendications 1 à 7 et un générateur de champ magnétique (1002, 1004, 1010) conçu pour fournir un champ magnétique (H) à variation temporelle dans ladite au moins une sonde (100 ; 100a ; 100b ; 100c ; 100d ; 100e ; 100f ; 100g) .

11. Système (1000) selon la revendication 10, dans lequel ledit générateur de champ magnétique (1002, 1004, 1010) est configuré pour fournir un champ magnétique (H) sensiblement statique pour aligner l'élément (120) ayant un moment de dipôle magnétique permanent dans une direction sélectionnée.

12. Système (1000) l'une des revendications 10 à 11, ledit système (1000) comprenant un récepteur (1012) configuré pour recevoir un flux de données en provenance de l'élément (120).
